(19) Europäisches Patentamt
European Patent Office
Office européen des brevets

(11) Veröffentlichungsnummer: **0 054 873**
**B1**

# (12) EUROPÄISCHE PATENTSCHRIFT

(45) Veröffentlichungstag der Patentschrift: **03.10.84**

(51) Int. Cl.³: **C 07 D 271/08,** A 61 K 31/42

(21) Anmeldenummer: **81110400.9**

(22) Anmeldetag: **12.12.81**

## (54) 3,4-Disubstituierte 1,2,5-Oxadiazol-2-oxide, Verfahren zu ihrer Herstellung und sie enthaltende pharmazeutische Zubereitungen.

(30) Priorität: **18.12.80 DE 3047749**

(43) Veröffentlichungstag der Anmeldung: **30.06.82 Patentblatt 82/26**

(45) Bekanntmachung des Hinweises auf die Patenterteilung: **03.10.84 Patentblatt 84/40**

(84) Benannte Vertragsstaaten: **AT BE CH DE FR GB IT LI NL SE**

(56) Entgegenhaltungen:
**CH - A - 498 856**
**CH - A - 601 276**

**Die Akte enthält technische Angaben, die nach dem Eingang der Anmeldung eingereicht wurden und die nicht in dieser Patentschrift enthalten sind.**

(73) Patentinhaber: **CASSELLA Aktiengesellschaft, Hanauer Landstrasse 526, D-6000 Frankfurt am Main 61 (DE)**

(72) Erfinder: **Schönafinger, Karl, Dr., Parkstrasse 1, D-8531 Uehlfeld (DE)**
Erfinder: **Beyerle, Rudi, Dr., An der Pfaffenmauer 44, D-6000 Frankfurt am Main 60 (DE)**
Erfinder: **Mogilev, Anton, Dr., Diaminstrasse 6, D-6000 Frankfurt am Main 61 (DE)**
Erfinder: **Bohn, Helmut, Dr., Kranzbergring 11, D-6369 Schöneck (DE)**
Erfinder: **Just, Melitta, Dr., Hüttenbergweg 6, D-6369 Schöneck (DE)**
Erfinder: **Martorana, Piero A., Dr., Kaiser-Friedrich-Promenade 108, D-6380 Bad Homburg (DE)**
Erfinder: **Nitz, Rolf-Eberhard, Dr., Heinrich-Bingemer-Weg 64, D-6000 Frankfurt am Main 60 (DE)**

(74) Vertreter: **Urbach, Hans-Georg, Dr. et al, Hanauer Landstrasse 526, D-6000 Frankfurt am Main 61 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 054 873 B1

**Beschreibung**

Die Erfindung betrifft 3,4-disubstituierte 1,2,5-Oxadiazol-2-oxide der Formel I

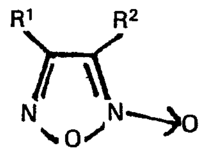

(I)

und ihre pharmakologisch annehmbaren Säureadditionsverbindungen, Verfahren zu ihrer Herstellung, diese Verbindungen zur Verwendung als pharmakologische Wirkstoffe und sie enthaltende pharmazeutische Zubereitungen.

In der Formel I steht einer der Reste $R^1$, $R^2$ für $-CH_3$ und der andere für

$-CONHR^3N(R^4)_2$, 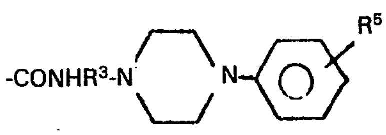

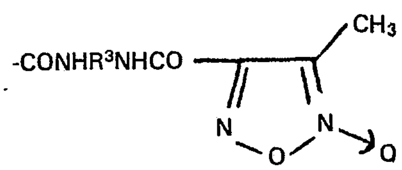

,

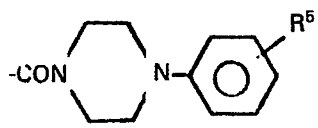

$-CONHR^3OR^6$, $-CONHR^7$, $-CONR^4R^8$,

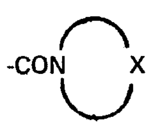

, 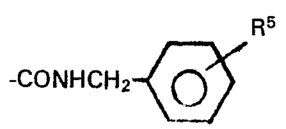

,

wobei

$R^3$ einen Alkylenrest $-C_nH_{2n}-$ mit n = 2, 3, oder 4 bedeutet,

$R^4$, $R^8$ = $-CH_3$, $-C_2H_5$,

$R^5$ = $-OCH_3$, -Cl,

$R^6$ = -H, $-CH_3$,

$R^7$ = Alkyl mit 1 bis 4 C-Atomen, Cycloalkyl mit 5 bis 7 C-Atomen, Allyl, Pyridyl-methyl, und

X = $-(CH_2)_2-O-(CH_2)_2-$, $-(CH_2)_2-\overset{CH_3}{N}-CH_2)_2-$, $-CH_2CH_2CH_2CH_2-$ oder $-CH_2CH_2CH_2CH_2CH_2-$ bedeuten.

Die für $R^7$ (und die für das nachstehend erwähnte $R^9$) stehenden Alkylreste und die für $R^3$ stehenden Alkylenreste können geradkettig oder verzweigt sein. $R^3$ bedeutet vorzugsweise $-CH_2CH_2-$ oder $-CH_2CH_2CH_2-$. Von den Verbindungen der Formel I sind diejenigen bevorzugt, bei denen $R^2$ = $-CH_3$ ist, ferner diejenigen, die im Rest $R^1$ eine -NH-Gruppierung besitzen.

Die Verbindungen der Formel I mit $R^2$ = $-CH_3$ können erfindungsgemäss durch Umsetzung von 3-Methyl-1,2,5-oxadiazol-2-oxid-4-carbonsäureestern der Formel II mit einem Amin HZ

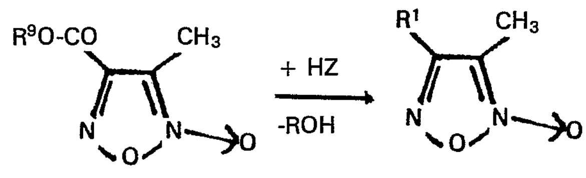


(II) (Ia)

hergestellt werden. $R^9$ bedeutet dabei einen Alkylrest mit 1 bis 6 C-Atomen, insbesondere mit 1 bis 4 C-Atomen, vorzugsweise Methyl oder Ethyl. Das Amin HZ wird dabei so ausgewählt, dass der Rest Z zusammen mit der Carbonylgruppe des Esters II in der Verbindung Ia den Rest $R^1$ bildet. Z hat daher die folgenden Bedeutungen:

$-NHR^3N(R^4)_2$, 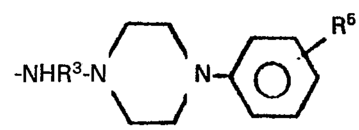

,

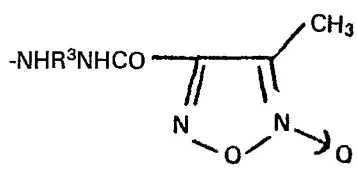

,

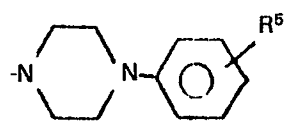

$-NHR^3OR^6$, $-NHR^7$, $-NR^4R^8$,

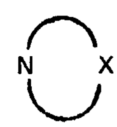

, 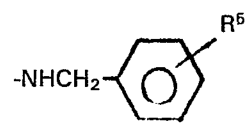

,

Die Umsetzung der Verbindung II mit dem Amin HZ wird zweckmässigerweise in einem geeigneten Lösungs- oder Dispergiermittel durchgeführt. Als derartige Lösungs- oder Dispergiermittel können beispielsweise Alkohole, wie Methanol, Ethanol, i-Propanol; ferner Ether, wie Diethylether, Dioxan, Tetrahydrofuran; Ketone wie Aceton; Kohlenwasserstoffe, wie Toluol, Xylole, Petrolether; halogenierte Kohlenwasserstoffe, wie Chloroform, Chlorbenzol; polare aprotische Lösungsmittel, wie z.B. Acetonitril, Dimethylformamid oder wässrige Lösungen des Amins HZ verwendet werden. Die Reaktion kann bei Temperaturen im Bereich von 0°C bis zur Rückflusstemperatur des Lösungs- oder Dispergiermittels durchgeführt werden, in der Regel erfolgt die Reaktion bei 15 - 25°C.

Die Verbindung der Formel II mit $R^9$ = Ethyl ist in

Berichte der deutschen chemischen Gesellschaft *28*, 2681 (1895) beschrieben als Ester der Peroxydiisonitrosobuttersäure. Verbindungen der Formel II mit anderen $R^9$-Resten lassen sich bei Wahl geeigneter Ausgangsprodukte analog herstellen.

Erfindungsgemässe Verbindungen mit $R^1 = CH_3$, denen die Formel Ib zukommt

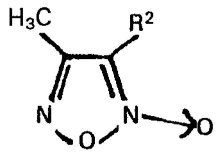

(Ib)

werden aus den Verbindungen Ia durch thermische Umlagerung hergestellt. Die thermische Umlagerung wird zweckmässigerweise ohne Lösungs- oder Dispergiermittel durch Erhitzen der Substanz auf Temperaturen von 150 bis 220°C, vorzugsweise 180 bis 200°C, durchgeführt. Die thermische Umlagerung ist in der Regel nach 15 bis 120 Minuten, in den meisten Fällen nach 30 bis 60 Minuten, beendet. Der Verlauf der Umlagerung kann dünnschichtchromatographisch verfolgt werden.

Die erfindungsgemässen Verbindungen I, die eine basische Seitenkette aufweisen, bilden mit anorganischen oder organischen Säuren Salze. Solche Säuren sind beispielsweise Chlorwasserstoff-, Bromwasserstoff-, Phosphor-, Schwefel-, Oxal-, Milch-, Wein-, Essig-, Salicyl-, Benzoe-, Zitronen-, Ascorbin-, Adipin- und Naphthalindisulfonsäure. Die Säureadditionsverbindungen werden in bekannter Weise durch Vereinigen der Komponenten in einem geeigneten Lösungs- oder Dispergiermittel erhalten.

Die Verbindungen der Formel I und ihre pharmakologisch annehmbaren Säureadditionsverbindungen besitzen wertvolle pharmakologische Eigenschaften. Besonders ausgeprägt ist ihre Wirkung auf das Herz-Kreislauf-System. Sie senken in niedriger Dosierung den Blutdruck, vermindern den peripheren Widerstand und führen über eine Senkung des Pulmonalarteriendrucks bei wenig beeinflusster Herzfrequenz zu einer Verminderung der Herzarbeit.

Die Verbindungen der Formel I und ihre pharmakologisch annehmbaren Säureadditionssalze können daher am Menschen als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der Formel I oder eines Säureadditionssalzes davon, neben üblichen pharmazeutisch zulässigen Träger- und Zusatzstoffen enthalten.

Die Heilmittel können oral, z.B. in Form von Pillen, Tabletten, Lacktabletten, Dragees, Hart- und Weichgelatinekapseln, Lösungen, Sirupen, Emulsionen oder Suspensionen oder Aerosolmischungen verabreicht werden. Die Verabreichung kann aber auch rektal, z.B. in Form von Suppositorien, oder parenteral, z.B. in Form von Injektionslösungen, oder perkutan, z.B. in Form von Salben oder Tinkturen, erfolgen.

Zur Herstellung der pharmazeutischen Präparate werden pharmazeutisch inerte anorganische oder organische Trägerstoffe verwendet. Für die Herstellung von Pillen, Tabletten, Dragees und Hartgelatinekapseln verwendet man z.B. Lactose, Maisstärke oder Derivate davon, Talk, Stearinsäure oder deren Salze usw. Trägerstoffe für Weichgelatinekapseln und Suppositorien sind z.B. Fette, Wachse, halbfeste und flüssige Polyole, natürliche oder gehärtete Öle usw. Als Trägerstoffe für die Herstellung von Lösungen und Sirupen eignen sich z.B. Wasser, Saccharose, Invertzucker, Glukose, Polyole usw. Als Trägerstoffe für die Herstellung von Injektionslösungen eignen sich z.B. Wasser, Alkohole, Glyzerin, Polyole, pflanzliche Öle usw.

Die pharmazeutischen Präprate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süss-, Färbe-, Geschmacks- oder Aromatisierungs-, Dikkungs-, Verdünnungs-Mittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der Formel I oder ihrer pharmakologisch annehmbaren Säureadditionssalze und noch andere therapeutisch wirksame Stoffe enthalten.

Derartige andere therapeutisch wirksame Substanzen sind beispielsweise: $\beta$-Rezeptorenblocker, wie z.B. Propranolol, Pindolol, Metoprolol; Vasodilatatoren, wie z.B. Carbochromen; Beruhigungsmittel, wie z.B. Barbitursäurederivate, 1,4-Benzodiazepine und Meprobamat; Diuretica, wie z.B. Chlorothiazid; das Herz tonisierende Mittel, wie z.B. Digitalispräparate; blutdrucksenkende Mittel, wie z.B. Hydralazin, Dihydralazin, Prazosin, Clonidin, Rauwolfia-Alkaloide; Mittel, die den Fettsäurespiegel im Blut senken, wie z.B. Bezafibrat, Fenofibrat; Mittel für die Thromboseprophylaxe, wie z.B. Phenprocoumon.

Die Verbindungen der Formel I, ihre pharmakologisch annehmbaren Säureadditionssalze und pharmazeutische Präparate, welche die Verbindungen der Formel I oder ihre pharmakologisch annehmbaren Säureadditionssalze als Wirkstoffe enthalten, können am Menschen bei der Bekämpfung bzw. Vorbeugung von Erkrankungen des kardiovaskulären Systems verwendet werden, beispielsweise als antihypertensive Heilmittel bei den verschiedenen Formen des Bluthochdrucks, bei der Bekämpfung bzw. Vorbeugung von Angina pectoris usw. Die Dosierung kann innerhalb weiter Grenzen variieren und ist in jedem einzelnen Fall den individuellen Gegebenheiten anzupassen. Im allgemeinen ist bei oraler Verabreichung pro menschlichem Individuum eine Tagesdosis von 0,2 bis 150 mg, vorzugsweise 1 bis 30 mg, angemessen. Auch bei anderen Applikationsformen liegt die Tagesdosis, wegen der guten Resorption der Wirkstoffe, in ähnlichen Mengenbereichen, d.h. im allgemeinen ebenfalls bei 0,2 bis 150 mg/Mensch. Die Tagesdosis wird normalerweise in mehrere, z.B. 2 bis 4 Teilverabreichungen aufgeteilt.

Die pharmazeutischen Zubereitungen enthalten von den Wirkstoffen der Formel I oder ihrer pharmakologisch annehmbaren Säureadditionssalze 0,1 bis 50 mg, vorzugsweise 0,5 bis 10 mg/Dosis.

Die Untersuchungen über die antianginöse und antihypertensive Wirkung der Verbindungen der Formel I wurden an Bastardhunden beiderlei Geschlechts in Pentobarbital-Narkose (30 bis 40 mg/kg i.v.) oder in Urethan-Chloralose-Narkose (3 ml /kg Urethan-Chloralose-Gemisch i.v. = 20 mg/kg Chloralose und 250 mg/kg Urethan) durchgeführt. Die Beatmung der Tiere erfolgte mit einem Bird-Mark-7-Respirator. Der endexpiratorische Kohlensäuregehalt (gemessen mit dem Uras) betrug zwischen 4,5 und 5 Vol.-%. Während des gesamten Versuchs erhielten die Tiere mit Pentobarbital-Narkose eine Dauerinfusion von Pentobarbital i.v. = 4 mg/kg/6 ml/h, um eine konstante Narkosetiefe zu gewährleisten; die Tiere mit Urethan-Chloralose-Narkose erhielten keine Dauerinfusion. Die Infusion wurde durch die Vena cephalica gegeben. Nach der Präparation des Versuchstieres wurde ca. 1 Stunde gewartet, bis sich alle haemodynamischen Parameter eingestellt hatten (steady state). Danach wurde mit dem eigentlichen Versuch begonnen.

Der systolische und diastolische Blutdruck wurde peripher in der Arteria femoralis über einen Statham-Druckaufnehmer gemessen. Ein über die Arteria carotis in den linken Ventrikel geschobener Millar-Tip-Kathether lieferte das Signal für den LVEDP (linksventrikulärer enddiastolischer Druck) und die Herzfrequenz. Mit einem zweiten, über die Vena jugularis eingeschobenen Tip-Katheter wurde der mittlere Blutdruck in der Arteria pulmonalis erfasst. Die erhaltenen Ergebnisse sind in der folgenden Tabelle angegeben.

| Substanz | Dosis mg/kg (i.v.) | LVEDP Δ mm/Hg | PAP Δ mm/Hg | BDm Δ mm/Hg | HF Δ/min |
|---|---|---|---|---|---|
| ISDN | 0,05 | —2,1 | —0,7 | —19 | ± 0 |
| A | 0,05 | —3 | —2 | —30 | ±10 |
| B | 0,1 | —2,5 | —1 | —25 | ± 0 |
| C | 0,05 | —2 | —1 | —35 | ± 0 |

In der Tabelle bedeuten:

| | | |
|---|---|---|
| LVEDP | = | linksventrikulärer enddiastolischer Druck |
| PAP | = | mittlerer Pulmonalarteriendruck |
| BDm | = | mittlerer peripherer Blutdruck |
| HF | = | Herzfrequenz |
| ISDN | = | Isosorbiddinitrat (Vergleichssubstanz) |
| A | = | 1,2,5-Oxadiazol-2-oxid-4-methyl-3--carbonsäure-methylamid |
| B | = | 1,2,5-Oxadiazol-2-oxid-3-methyl-4--carbonsäure-methoxiethylamid |
| C | = | 1,2,5-Oxadiazol-2-oxid-3-methyl-4--carbonsäure-(pyrid-3-yl-methyl-amid) |

Aus der CH-A-601 276 ist ein Verfahren zur Herstellung von Furoxanderivaten bekannt, die jedoch in 3,4-Stellung anders substituiert sind, als die erfindungsgemässen Furoxanderivate. Eine Verwendung für die bekannten Furoxanderivate ist nicht angegeben. Die vorliegende Erfindung wird durch diesen Stand der Technik nicht nahegelegt, ebensowenig durch die CH-A-498 856, die ein Verfahren zur Herstellung von 3-Amino-4-phenyl-furazanderivaten betrifft, die im Phenylkern ein-, zwei- oder dreifach substituiert sind und die zur Beruhigung von schwachen Erregungszuständen und zur Behebung der Muskelsteife verwendet werden.

Die folgenden Beispiele dienen der weiteren Erläuterung der Erfindung.

*Beispiel 1*

*1,2,5-Oxadiazol-2-oxid-3-methyl-4-(carbonsäure-2-hydroxiethylamid)*

19,2 g 1,2,5-Oxadiazol-2-oxid-3-methyl-4-carbonsäureethylester werden in 50 ml Ethanol gelöst. Bei 20°C wird eine Lösung von 6,1 g Ethanolamin in 40 ml Ethanol zugetropft. Die Mischung wird 4 Stunden bei 20°C gerührt, auf 0°C abgekühlt. Der Feststoff wird abgesaugt, mit wenig kaltem Ethanol gewaschen und aus Isopropanol umkristallisiert: Farblose Kristalle vom Fp 107 bis 108°C.

Analog diesem Verfahren können die folgenden Verbindungen in den angegebenen Lösungsmitteln und bei den angegebenen Reaktionstemperaturen hergestellt werden (NDS = Naphthalin-1,5-disulfonsäure):

1,2,5-Oxadiazol-2-oxid-3-methyl-4-carbonsäure-allylamid (Öl) in Methanol bei 15°C.

1,2,5-Oxadiazol-2-oxid-3-methyl-4-carbonsäure-methylamid (Fp 89 bis 91°C) in Wasser bei 0°C.

1,2,5-Oxadiazol-2-oxid-3-methyl-4-carbonsäure-ethylamid (Fp 80 bis 82°C) in i-Propanol bei 20°C.

1,2,5-Oxadiazol-2-oxid-3-methyl-4-carbonsäure-isopropylamid (Fp 94 bis 95°C) in Toluol bei 50°C.

1,2,5-Oxadiazol-2-oxid-3-methyl-4-carbonsäure-(2-diethylaminoethylamid (Fp 216°C, NDS-Salz) in Diethylether bei Rückflusstemperatur.

1,2,5-Oxadiazol-2-oxid-3-methyl-4-carbonsäure-(3-diethylaminopropylamid) (Öl) in Diethylether bei Rückflusstemperatur.

1,2,5-Oxadiazol-2-oxid-3-methyl-4-carbonsäure-[3-(4-o-methoxiphenylpiperazin-1-yl)-propylamid] (Fp 232°C, NDS-Salz) in Toluol bei 110°C.

1,2,5-Oxadiazol-2-oxid-3-methyl-4-carbonsäure-(4-o-methoxiphenylpiperazinid) (Fp 152 bis 153°C) in Chlorbenzol bei 100°C.

1,2,5-Oxadiazol-2-oxid-3-methyl-4-carbonsäure-[3-(4-o-chlorphenylpiperazin-1-yl)-propylamid] (Fp 205 bis 206°C Zers. NDS-Salz) in Toluol bei 110°C.

1,2,5-Oxadiazol-2-oxid-3-methyl-4-carbonsäure-[3-(4-o-methoxiphenylpiperazin-1-yl)-2-methyl--propylamid] (Fp 114 bis 115°C) in Toluol bei 110°C.

1,2,5-Oxadiazol-2-oxid-3-methyl-4-carbonsäure-cyclopentylamid (Fp 104 bis 106°C) in Ethanol bei 40°C.

1,2,5-Oxadiazol-2-oxid-3-methyl-4-carbonsäure-cyclohexylamid (Fp 95 bis 96,5°C) in Ethanol bei 60°C.

1,2,5-Oxadiazol-2-oxid-3-methyl-4-carbonsäure-(2-methoxiethylamid) (Fp 69 bis 70°C) in Ethanol bei 20°C.

1,2-Bis-[1,2,5-oxadiazol-2-oxid-3-methyl-4-carbonylamino]-ethan (Fp 195 bis 200°C Zers.) in Ethanol bei 25°C.

1,3-Bis-[1,2,5-oxadiazol-2-oxid-3-methyl-4-carbonylamino]-propan (Fp 183 bis 186°C) in Ethanol bei 25°C.

1,2,5-Oxadiazol-2-oxid-3-methyl-4-carbonsäure-morpholid (Fp 75 bis 78°C) in Acetonitril bei 40°C.

1,2,5-Oxadiazol-2-oxid-3-methyl-4-carbonsäure-benzylamid (Fp 91 bis 93°C) in Petrolether bei 20°C.

1,2,5-Oxadiazol-2-oxid-3-methyl-4-carbonsäure-pyrrolidid (Fp 65 bis 67°C) in Ethanol bei 15°C.

1,2,5-Oxadiazol-2-oxid-3-methyl-4-carbonsäure-(4-methylpiperazid)-hydrochlorid (Fp 278 bis 280°C Zers.) in Methylenchlorid bei 25°C.

1,2,5-Oxadiazol-2-oxid-3-methyl-4-carbonsäure-dimethylamid (Fp bis 72°C) in Dimethylformamid/Wasser bei 30°C.

1,2,5-Oxadiazol-2-oxid-3-methyl-4-carbonsäure-(pyrid-3-yl-methylamid) (Fp 127 bis 129°C) in Ethanol bei 25°C.

*Beispiel 2*

*1,2,5-Oxadiazol-2-oxid-4-methyl-3-carbonsäure-methylamid*

5 g 1,2,5-Oxadiazol-2-oxid-4-methyl-3-carbonsäure-methylamid werden ohne Lösungsmittel 30 Minuten auf 190°C erwärmt. Nach Abkühlen der Schmelze wird das Produktgemisch säulenchromatographisch (Laufmittel: Methylenchlorid, Säulenfüllung: Kieselgel) aufgetrennt. Die schneller laufende Fraktion wird eingeengt und mit Ligroin verrührt. Der weisse Feststoff wird abgesaugt und ist nach Trocknung im Vakuum analysenrein: Fp 57 bis 59°C.

Analog diesem Verfahren können die folgenden Verbindungen bei den nach den Verbindungen angegebenen Reaktionstemperaturen hergestellt werden:

1,2,5-Oxadiazol-2-oxid-4-methyl-3-carbonsäure-ethylamid (Öl) 150°C,

1,2,5-Oxadiazol-2-oxid-4-methyl-3-carbonsäure-(2-methoxiethylamid) (Öl) bei 220°C,

1,2,5-Oxadiazol-2-oxid-4-methyl-3-carbonsäure-isopropylamid (Fp 65 bis 68°C) bei 200°C,

1,2,5-Oxadiazol-2-oxid-4-methyl-3-carbonsäure-(2-diethylaminoethylamid) (Fp 200 bis 202°C NDS-Salz) bei 190°C,

1,2,5-Oxadiazol-2-oxid-4-methyl-3-carbonsäure-(4-o-methoxiphenylpiperazinid) (Fp 129 bis 131°C) bei 200°C,

1,2,5-Oxadiazol-2-oxid-4-methyl-3-carbonsäure-cyclopentylamid (Fp 91 bis 94°C) bei 190°C,

1,2,5-Oxadiazol-2-oxid-4-methyl-3-carbonsäure-morpholid (Fp 45 bis 46°C) bei 185°C,

1,2,5-Oxadiazol-2-oxid-4-methyl-3-carbonsäure-pyrrolidid (Öl) bei 180°C,

1,2,5-Oxadiazol-2-oxid-4-methyl-3-carbonsäure-(4-methylpiperazid) (Fp des Hydrochlorids 250 bis 252°C Zers.) bei 195°C.

Die Abkürzung «Zers.» bedeutet Zersetzung. Die Angabe «NDS-Salz» im Zusammenhang mit dem Schmelzpunkt bei einigen der vorgenannten Verbindungen bedeutet, dass es sich um den Schmelzpunkt der Additionsverbindung mit der Naphthalin-1,5-disulfonsäure handelt.

Die Strukturen der synthetisierten Verbindungen können durch die Elementaranalyse und durch die IR- und NMR-Spektren bestätigt werden.

**Patentansprüche für die Vertragsstaaten:**
BE, CH, DE, FR, GB, IT, LI, NL, SE

1. 3,4-Disubstituierte 1,2,5-Oxadiazol-2-oxide der Formel I

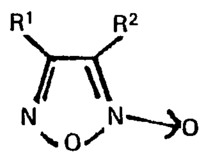

(I)

worin
einer der Reste $R^1$, $R^2$ für $-CH_3$ und der andere für

$-CONHR^3N(R^4)_2$, 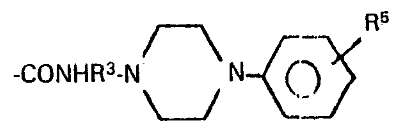

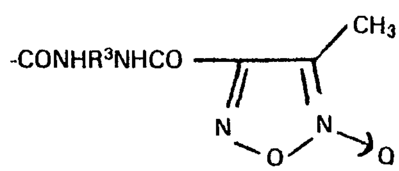 ,

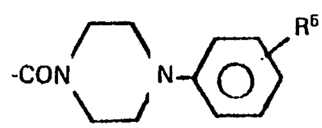

$-CONHR^3OR^6$, $-CONHR^7$, $-CONR^4R^8$,

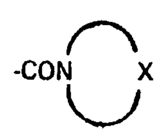 , 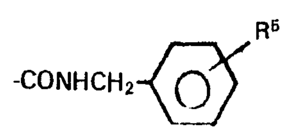 ,

steht, wobei

$R^3$ einen Alkylenrest $-C_nH_{2n}-$ mit n = 2, 3, oder 4 bedeutet,

$R^4$, $R^8$ = $-CH_3$, $-C_2H_5$,

$R^5$ = $-OCH_3$, -Cl,

$R^6$ = -H, $-CH_3$,

$R^7$ = Alkyl mit 1 bis 4 C-Atomen, Cycloalkyl mit 5 bis 7 C-Atomen, Allyl, Pyridyl-methyl, und

X = $-(CH_2)_2-O-(CH_2)_2-$, $-(CH_2)_2-\overset{CH_3}{\overset{|}{N}}-CH_2)_2-$, $-CH_2CH_2CH_2CH_2-$ oder $-CH_2CH_2CH_2CH_2CH_2-$ bedeuten, sowie ihre pharmakologisch annehmbaren Säureadditionsverbindungen.

2. 3,4-Disubstituierte 1,2,5-Oxadiazol-2-oxide nach Anspruch 1, dadurch gekennzeichnet, dass einer der Reste $R^1$, $R^2$ für $CH_3$ und der andere für

$-CONHR^3N(R^4)_2$, 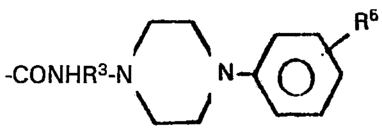

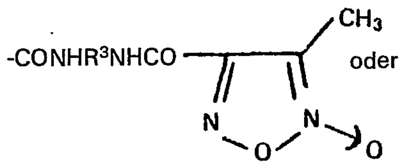 oder $-CONHR^3OR^6$

steht, wobei
$R^3$ = $-CH_2CH_2-$ oder $-CH_2CH_2CH_2-$ bedeutet und
$R^4$, $R^5$ und $R^6$ die in Anspruch 1 angegebenen Bedeutungen besitzen.

3. 3,4-Disubstituierte 1,2,5-Oxadiazol-2-oxide nach Anspruch 1 oder 2, dadurch gekennzeichnet, dass sie eine -NH-Gruppierung enthalten.

4. Verfahren zur Herstellung von Verbindungen der Formel Ia

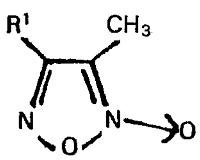

(Ia)

eines oder mehrerer Ansprüche 1 bis 3, dadurch gekennzeichnet, dass ein 3-Methyl-1,2,5-oxadiazol-2-oxid-4-carbon-säureester der Formel II

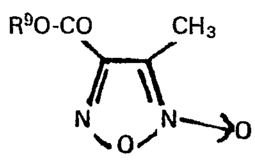

(II)

worin $R^9$ Alkyl mit 1 bis 6 C-Atomen bedeutet, mit einem Amin HZ umgesetzt wird und dabei das Amin so ausgewählt wird, dass der Rest Z mit der Carbonylgruppe des Esters II den Rest $R^1$ bildet und die erhaltene Verbindung gegebenenfalls in eine Säureadditionsverbindung überführt wird.

5. Verfahren nach Anspruch 4, dadurch gekennzeichnet, dass die Umsetzung in einem Lösungs- oder Dispergiermittel bei Temperaturen von 0°C bis zur Rückflusstemperatur des Lösungs- oder Dispergiermittels durchgeführt wird.

6. Verfahren zur Herstellung der Verbindungen der Formel Ib

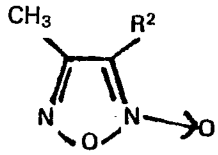

(Ib)

eines oder mehrerer Ansprüche 1 bis 3, dadurch gekennzeichnet, dass eine Verbindung der Formel Ia

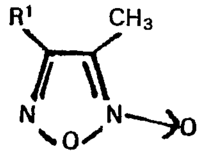

(Ia)

auf Temperaturen von 150 bis 220°C, vorzugsweise 180 bis 200°C, erhitzt wird und die erhaltene Verbindung gegebenenfalls in eine Säureadditionsverbindung überführt wird.

7. 3,4-Disubstituierte 1,2,5-Oxadiazol-2-oxide eines oder mehrerer Ansprüche 1 bis 3 oder ihre pharmakologisch annehmbaren Säureadditionsverbindungen zur Verwendung bei der Bekämpfung oder Vorbeugung von Erkrankungen des kardiovaskulären Systems.

8. Pharmazeutische Zubereitung, enthaltend eine wirksame Dosis eines 3,4-disubstituierten 1,2,5-Oxadiazol-2-oxids nach einem oder mehreren der Ansprüche 1 bis 3 oder einer ihrer pharmakologisch annehmbaren Säureadditionsverbindungen neben pharmazeutisch zulässigen Träger- und gegebenenfalls Zusatzstoffen und gegebenenfalls noch anderen therapeutisch wirksamen Substanzen.

**Patentansprüche für den Vertragsstaat:** AT

1. Verfahren zur Herstellung von 3,4-disubstituierten 1,2,5-Oxadiazol-2-oxiden der Formel I

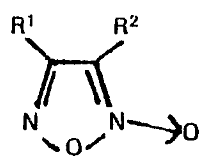

(I)

worin
einer der Reste $R^1$, $R^2$ für $-CH_3$ und der andere für

$-CONHR^3N(R^4)_2$, 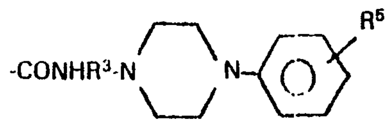

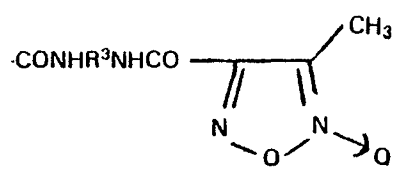 ,

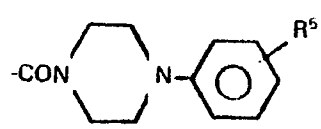

$-CONHR^3OR^6$, $-CONHR^7$, $-CONR^4R^8$,

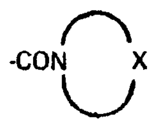 , 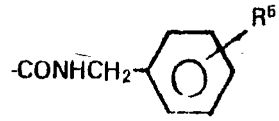 ,

steht, wobei

$R^3$ einen Alkylenrest $-C_nH_{2n}-$ mit n = 2, 3, oder 4 bedeutet,

$R^4$, $R^8$ = $-CH_3$, $-C_2H_5$,

$R^5$ = $-OCH_3$, $-Cl$,

$R^6$ = $-H$, $-CH_3$,

$R^7$ = Alkyl mit 1 bis 4 C-Atomen, Cycloalkyl mit 5 bis 7 C-Atomen, Allyl, Pyridyl-methyl, und

X = $-(CH_2)_2-O-(CH_2)_2-$, $-(CH_2)_2-N(CH_3)-CH_2)_2-$, $-CH_2CH_2CH_2CH_2-$ oder $-CH_2CH_2CH_2CH_2CH_2-$ bedeuten, sowie ihre pharmakologisch annehmbaren Säureadditionsverbindungen, dadurch gekennzeichnet, dass zur Herstellung von Verbindung der Formel Ia

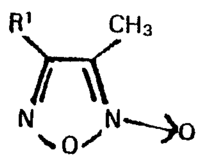

(Ia)

ein 3-Methyl-1,2,5-oxadiazol-2-oxid-4-carbon-säureester der Formel II

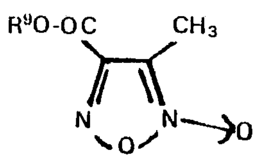

(II)

worin $R^9$ Alkyl mit 1 bis 6 C-Atomen bedeutet, mit einem Amin HZ umgesetzt wird und dabei das Amin so ausgewählt wird, dass der Rest Z mit der Carbonylgruppe des Esters II den Rest $R^1$ bildet und die erhaltene Verbindung gegebenenfalls in eine Säureadditionsverbindung überführt wird oder gegebenenfalls zur Herstellung der Verbindungen der Formel Ib

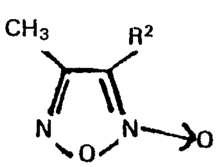 (Ib)

auf Temperaturen von 150 bis 220°C, vorzugsweise 180 bis 200°C, erhitzt wird und die erhaltene Verbindung gegebenenfalls in eine Säureadditionsverbindung überführt wird.

2. Verfahren nach Anspruch 1, dadurch gekennzeichnet, dass die Umsetzung des Esters der Formel II mit dem Amin HZ in einem Lösungs- oder Dispergiermittel bei Temperaturen von 0°C bis zur Rückflusstemperatur des Lösungs- und Dispergiermittels durchgeführt wird.

3. Verfahren nach den Ansprüchen 1 oder 2, dadurch gekennzeichnet, dass $R^9$ Alkyl mit 1 bis 4 C-Atomen, vorzugsweise Methyl oder Ethyl, bedeutet.

4. Verfahren nach einem oder mehreren der Ansprüche 1 bis 3, dadurch gekennzeichnet, dass das Amin HZ so ausgewählt wird, dass es mit der Carbonylgruppe des Esters II einen Rest $R^1$ bildet, der eine -NH-Gruppierung enthält.

5. Verfahren nach einem oder mehreren der Ansprüche 1 bis 4, dadurch gekennzeichnet, dass das Amin HZ so ausgewählt wird, dass es mit der Carbonylgruppe des Esters II einen Rest $R^1$ der Formel

$-CONHR^3N(R^4)_2$, 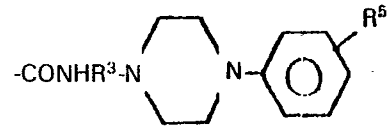

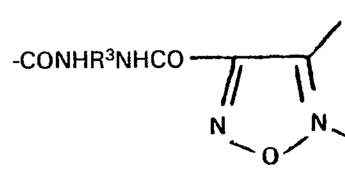 oder $-CONHR^3OR^6$

bildet, wobei

$R^3$ = $-CH_2CH_2-$ oder $-CH_2CH_2CH_2-$ bedeutet und $R^4$, $R^5$ und $R^6$ die in Anspruch 1 angegebenen Bedeutungen besitzen.

6. Verfahren zur Herstellung von Mitteln zur Bekämpfung oder Vorbeugung von Erkrankungen des kardiovaskulären Systems, dadurch gekennzeichnet, dass die 3,4-disubstituierten 1,2,5-Oxadiazol-2-oxide der Formel I sowie ihre pharmakologisch annehmbaren Säureadditionsverbindungen

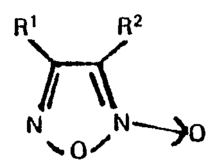 (I)

entsprechend Anspruch 1 hergestellt und die so erhaltenen Verbindungen der Formel I oder ihre pharmakologisch annehmbaren Säureadditionsverbindungen durch Mischen mit pharmazeutsich zulässigen Träger- und gegebenenfalls Zusatzstoffen in eine zur Verabreichung und Bekämpfung oder Vorbeugung von Erkrankungen des kardiovaskulären Systems geeignete Form gebracht werden.

**Revendications pour les Etats contractants:**
BE, CH, DE, FR, GB, IT, LI, NL, SE

1. Oxadiazole-1,2,5 oxydes-2 disubstitués en 3,4 qui répondent à la formule I

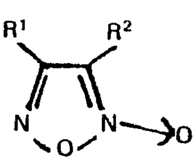 (I)

dans laquelle l'un des symboles $R^1$ et $R^2$ représente un radical $-CH_3$ et l'autre représente un radical répondant à l'une des formules suivantes:

$-CONHR^3N(R^4)_2$, 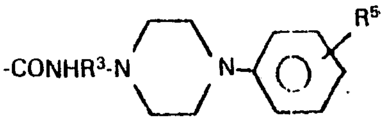

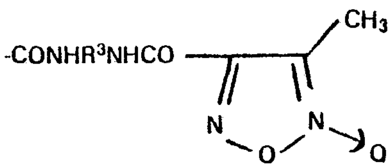,

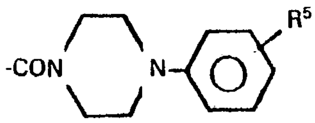

$-CONHR^3OR^6$, $-CONHR^7$, $-CONR^4R^8$,

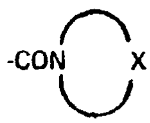, 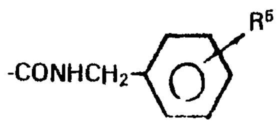,

dans lesquelles

$R^3$ représente un radical alkylène $-C_nH_{2n}-$ dont l'indice n est égal à 2, à 3 ou à 4,

$R^4$ et $R^8$ représentent chacun $-CH_3$ ou $-C_2H_5$,

$R^5$ représente $-OCH_3$ ou -Cl,

$R^6$ représente -H ou $-CH_3$,

$R^7$ représente un radical alkyle contenant de 1 à 4 atomes de carbone, un radical cycloalkyle contenant de 5 à 7 atomes de carbone, ou un radicale allyle ou pyridylméthyle, et

X représente un radical $-(CH_2)_2-O-(CH_2)_2-$, $-(CH_2)_2-N(CH_3)-CH_2)_2-$ $-CH_2CH_2CH_2CH_2-$ ou $-CH_2CH_2CH_2CH_2CH_2-$

et leurs composés d'addition d'acides acceptables du point de vue pharmacologique.

2. Oxadiazole-1,2,5 oxydes-2 disubstitués en 3,4 selon la revendication 1, caractérisés en ce que l'un des symboles $R^1$ et $R^2$ représente un radical $-CH_3$ et l'autre représente un radical répondant à l'une des formules suivantes:

$-CONHR^3N(R^4)_2$, 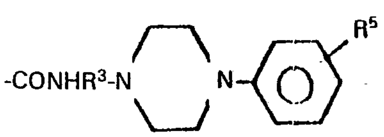

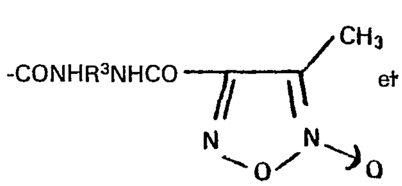 et $-CONHR^3OR^6$

dans lesquelles $R^3$ représente $-CH_2CH_2-$ ou $-CH_2CH_2CH_2-$ et $R^4$, $R^5$ et $R^6$ ont les significations données à la revendication 1.

3. Oxadiazole-1,2,5 oxydes-2 disubstitués en 3,4 selon l'une des revendications 1 et 2, caractérisés en ce qu'ils contiennent un radical -NH.

4. Procédé de préparation de composés selon l'une quelconque des revendications 1 à 3 et qui répondent à la formule Ia

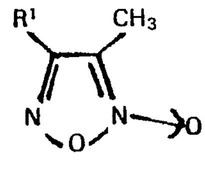

(Ia)

procédé caractérisé en ce qu'on fait réagir un ester d'acide méthyl-3 oxadiazole-1,2,5 oxyde-2 carboxylique-4 répondant à la formule II

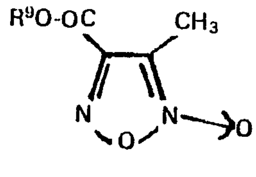

(II)

dans laquelle $R^9$ représente un radical alkyle contenant de 1 à 6 atomes de carbone, avec une amine HZ choisie de telle façon que le radical Z forme le radical $R^1$ avec le radical carbonyle de l'ester (II), et on transforme éventuellement le composé obtenu en un composé d'addition d'acide.

5. Procédé selon la revendication 4, caractérisé en ce qu'on effectue la réaction dans un solvant ou un milieu de dispersion à des températures comprises entre 0°C et la température de reflux du solvant ou milieu de dispersion.

6. Procédé de préparation de composés selon l'une quelconque des revendications 1 à 3 et qui répondent à la formule Ib

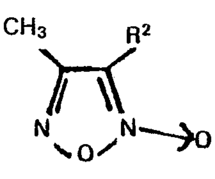

(Ib)

procédé caractérisé en ce qu'on chauffe un composé de formule Ia

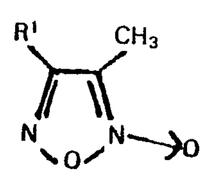

(Ia)

à des températures de 150 à 220°C, de préférence de 180 à 200°C, et on transforme éventuellement le composé obtenu en un composé d'addition d'acide.

7. Oxadiazole-1,2,5 oxydes-2 disubstitués en 3,4 selon l'une quelconque des revendications 1 à 3, ou leurs composés d'addition d'acides acceptables du point de vue pharmacologique, pour l'utilisation dans la lutte contre des maladies du système cardiovasculaire ou pour la prévention de ces maladies.

8. Médicament qui contient une dose efficace d'un oxadiazole-1,2,5 oxyde-2 disubstitué en 3,4, selon l'une quelconque des revendications 1 à 3, ou un de leurs composés d'addition d'acides acceptables du point de vue pharmacologique, avec, en plus des excipients et éventuellement des additifs permis en pharmacie et éventuellement aussi d'autres composés doués d'une activité thérapeutique.

**Revendications pour l'Etat contractant:** AT

1. Procédé de préparation d'oxadiazole-1,2,5 oxydes-2 disubstitués en 3,4 qui répondent à la formule I

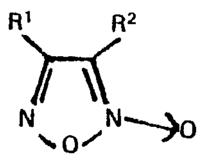

(I)

dans laquelle l'un des symboles $R^1$ et $R^2$ représente un radical $-CH_3$ et l'autre représente un radical répondant à l'une des formules suivantes:

$-CONHR^3N(R^4)_2$,

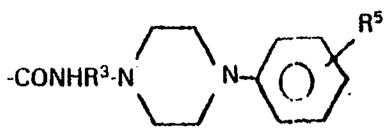

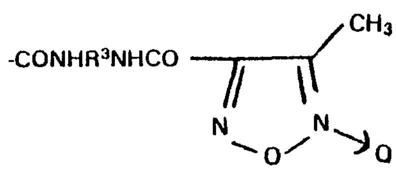

,

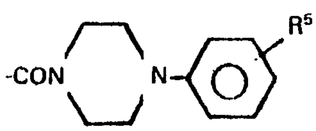

$-CONHR^3OR^6$, $-CONHR^7$, $-CONR^4R^8$,

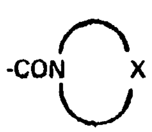

,

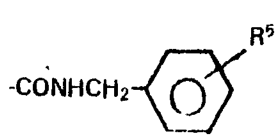

,

dans lesquelles

$R^3$ représente un radical alkylène $-C_nH_{2n}-$ don l'indice n est égal à 2, à 3 ou à 4,

$R^4$ et $R^8$ représentent chacun $-CH_3$ ou $-C_2H_5$,

$R^5$ représente $-OCH_3$ ou -Cl,

$R^6$ représente -H ou $-CH_3$,

$R^7$ représente un radical alkyle contenant de 1 à 4 atomes de carbone, un radical cycloalkyle contenant de 5 à 7 atomes de carbone, ou un radical allyle ou pyridylméthyle, et

X représente un radical $-(CH_2)_2-O-(CH_2)_2-$,

$CH_3$

$-(CH_2)_2-N-CH_2)_2-$ $-CH_2CH_2CH_2CH_2-$ ou $-CH_2CH_2CH_2CH_2CH_2-$

et de leurs composés d'addition d'acides acceptables du point de vue pharmacologique, procédé caractérisé en ce que, pour préparer des composés de formule Ia

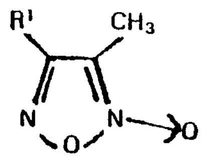

(Ia)

on fait réagir un ester d'acide méthyl-3 oxadiazole-1,2,5 oxyde-2 carboxylique-4 répondant à la formule II

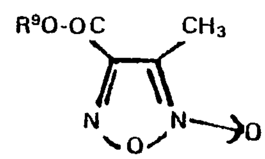

(II)

dans laquelle $R^9$ représente un radical alkyle contenant de 1 à 6 atomes de carbone, avec une amine HZ, l'amine étant alors choisie de telle façon que le radical Z forme le radical $R^1$ avec le radical carbonyle de l'ester (II), et on transforme éventuellement le composé obtenu en un composé d'addition d'acides ou, lorsqu'on veut préparer des composés de formule Ib

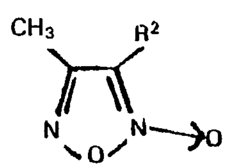

(Ib)

on chauffe (Ia) à des températures comprises entre 150 et 220 °C, de préférence entre 180 et 200 °C, et on transforme éventuellement le composé obtenu en un composé d'addition d'acide.

2. Procédé selon la revendication 1, caractérisé en ce que la réaction de l'ester de formule II avec l'amine HZ est effectuée dans un solvant ou un milieu de dispersion à des températures comprises entre 0 °C et la température de reflux du solvant ou du milieu de dispersion.

3. Procédé selon l'une des revendications 1 et 2, caractérisé en ce que $R^9$ représente un radical alkyle contenant de 1 à 4 atomes de carbone, de préférence un radical méthyle ou éthyle.

4. Procédé selon l'une quelconque des revendications 1 à 3, caractérisé en ce qu'on choisit l'amine HZ de telle façon qu'elle forme, avec le radical carbonyle de l'ester (II), un radical $R^1$ contenant un groupe -NH-.

5. Procédé selon l'une quelconque des revendications 1 à 4, caractérisé en ce qu'on choisit l'amine HZ de telle façon qu'elle forme, avec le radical carbonyle de l'ester (II), un radical $R^1$ répondant à l'une des formules suivantes:

$-CONHR^3N(R^4)_2$, 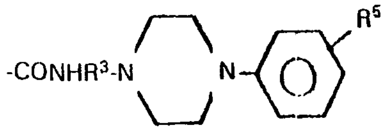

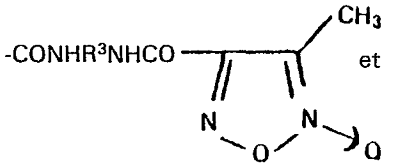 et $-CONHR^3OR^6$

dans lesquelles $R^3$ représente $-CH_2CH_2-$ ou $-CH_2CH_2CH_2-$ et $R^4$, $R^5$ et $R^6$ ont les significations données à la revendication 1.

6. Procédé de préparation de produits permettant de combattre ou de prévenir des maladies du système cardiovasculaire, procédé caractérisé en ce qu'on prépare selon la revendication 1 les oxadiazole-1,2,5 oxydes-2 disubstitués en 3,4 de formule I

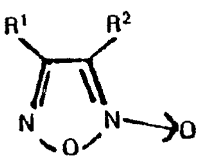 (I)

ainsi que leurs composés d'addition d'acides acceptables du point de vue pharmacologique, et on met les composés de formule I ainsi obtenus, ou leurs composés d'addition d'acides acceptables du point de vue pharmacologique, en les mélangeant avec des excipients et éventuellement avec des additifs permis en pharmacie, sous une forme qui convient pour l'administration et permet de combattre ou de prévenir des maladies du système cardiovasculaire.

**Claims for the contracting States:**
BE, CH, DE, FR, GB, IT, LI, NL, SE

1. 3,4-Disubstituted 1,2,5-oxadiazole-2-oxides of the formula I

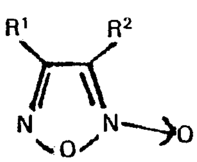 (I)

wherein one of the radicals $R^1$ and $R^2$ denotes $-CH_3$ and the other denotes

$-CONHR^3N(R^4)_2$, 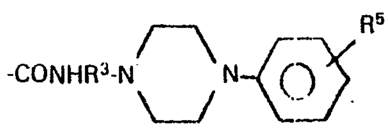

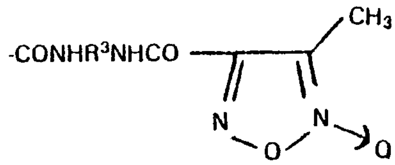 ,

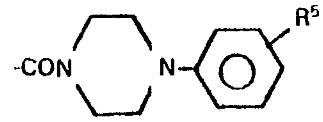

$-CONHR^3OR^6$, $-CONHR^7$, $-CONR^4R^8$,

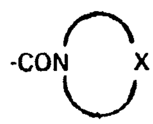 , 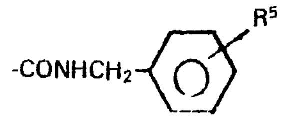 ,

wherein $R^3$ denotes an alkylene radical $-C_nH_{2n}-$, wherein n = 2, 3 or 4, $R^4$ and $R^8$ denote $-CH_3$ or $-C_2H_5$, $R^5$ denotes $-OCH_3$ or -Cl, $R^6$ denotes -H or $-CH_3$, $R^7$ denotes alkyl having 1 to 4 C atoms, cycloalkyl having 5 to 7 C atoms, allyl, or pyridyl methyl, and X denotes

$-(CH_2)_2-O-(CH_2)_2-$, $-(CH_2)_2-N(CH_3)-CH_2)_2-$ $-CH_2CH_2CH_2CH_2-$ or $-CH_2CH_2CH_2CH_2CH_2-$

and pharmacologically acceptable acid addition compounds thereof.

2. 3,4-Disubstituted 1,2,5-oxadiazole-2-oxides according to claim 1, characterised in that one of the radicals $R^1$ and $R^2$ denotes $-CH_3$ and the other denotes

$-CONHR^3N(R^4)_2$, 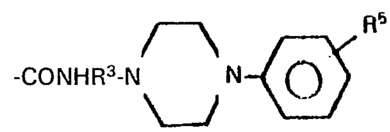

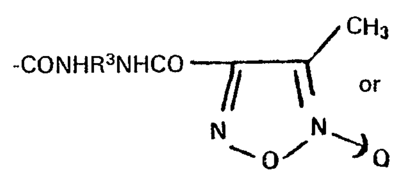 or $-CONHR^3OR^6$

wherein $R^3$ denotes $-CH_2CH_2-$ or $-CH_2CH_2CH_2-$ and $R^4$, $R^5$ and $R^6$ have the meanings given in claim 1.

3. 3,4-Disubstituted 1,2,5-oxadiazole-2-oxides according to claim 1 or 2, characterised in that they contain an -NH- grouping.

4. Process for the preparation of compounds of the formula Ia

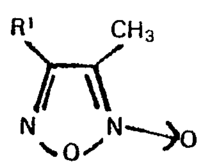

(Ia)

of one or several of claims 1 to 3, characterised in that a 3-methyl-1,2,5-oxadiazole-2-oxide-4--carboxylic acid ester of the formula II

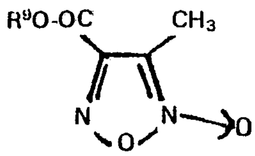

(II)

wherein $R^9$ denotes alkyl, having 1 to 6 C atoms, is reacted with an amine HZ and the amine employed here is selected in such a way that the radical Z, together with the carbonyl group of the ester II, forms the radical $R^1$, and the resulting compound is, if appropriate, converted into an acid addition compound.

5. Process according to claim 4, characterised in that the reaction is carried out in a solvent or dispersing agent at temperatures from 0°C to the reflux temperatures of the solvent or dispersing agent.

6. Process for the preparation of the compounds of the formula Ib

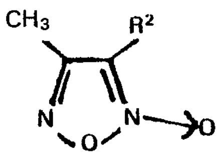

(Ib)

of one or several of claims 1 to 3, characterised in that a compound of the formula Ia

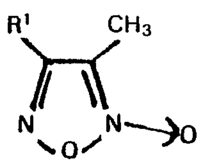

(Ia)

is heated to temperatures from 150 to 220°C, preferably 180 to 200°C, and the resulting compound is, if appropriate, converted into an acid addition compound.

7. 3,4-Disubstituted 1,2,5-oxadiazole-2-oxides of one or several of claims 1 to 3, or pharmaceutically acceptable acid addition compounds thereof, for use in combating or preventing diseases of the cardiovascular system.

8. Pharmaceutical formulation containing an effective dose of a 3,4-disubstituted 1,2,5-oxadiazole-2-oxide of one or several of claims 1 to 3, or of a pharmacologically acceptable acid addition compound thereof, together with pharmaceutically permissible excipient and, if appropriate, additives and, if appropriate, also other therapeutically acitve substances.

**Claims for the contracting State:** AT

1. Process for the preparation of 3,4-disubstituted 1,2,5-oxadiazole-2-oxides of the formula I

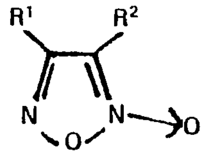

(I)

wherein one of the radicals $R^1$ and $R^2$ denotes $-CH_3$ and the other denotes

$-CONHR^3N(R^4)_2$, 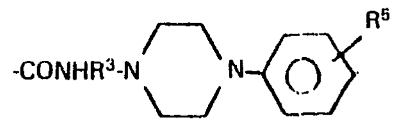

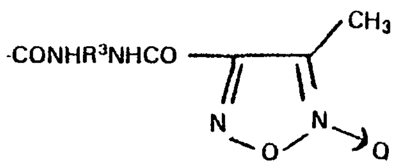

,

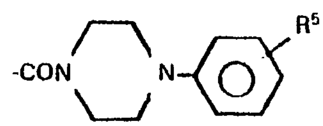

$-CONHR^3OR^6$, $-CONHR^7$, $-CONR^4R^8$,

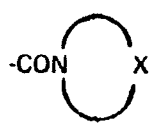 , 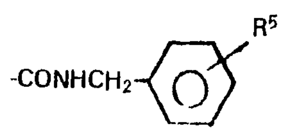 ,

wherein $R^3$ denotes an alkylene radical $-C_nH_{2n}-$, wherein n = 2, 3 or 4, $R^4$ and $R^8$ denote $-CH_3$ or $-C_2H_5$, $R^5$ denotes $-OCH_3$ or -Cl, $R^6$ denotes -H or $-CH_3$, $R^7$ denotes alkyl having 1 to 4 C atoms, cycloalkyl having 5 to 7 C atoms, allyl, or pyridyl methyl, and X denotes

$CH_3$

$-(CH_2)_2-O-(CH_2)_2-$, $-(CH_2)_2-N-CH_2)_2-$ $-CH_2CH_2CH_2-CH_2-$ or $-CH_2CH_2CH_2CH_2CH_2-$

as well as pharmacologically acceptable acid addition compounds thereof, characterised in that for the preparation of compounds of the formula Ia

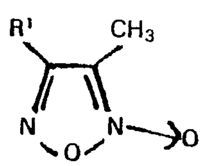

(Ia)

a 3-methyl-1,2,5-oxadiazole-2-oxide-4-carboxylic acid ester of the formula II

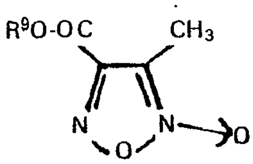

(II)

wherein $R^9$ denotes alkyl having 1 to 6 C atoms, is reacted with an amine HZ and the amine employed here is selected in such a way that the radical Z, together with the carbonyl group of the ester II, forms the radical $R^1$, and the resulting compound is, if appropriate, converted into an acid addition compound or, if appropriate, for the preparation of the compounds of the formula Ib

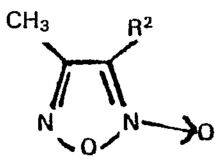

(Ib)

is heated to temperatures from 150 to 220°C, preferably 180 to 200°C, and the resulting compound is, if appropriate, converted into an acid addition compound.

2. Process according to claim 1, characterised in that the reaction of the ester of formula II with the amine HZ is carried out in a solvent or dispersing agent at temperatures from 0°C to the reflux temperature of the solvent or dispersing agent.

3. Process according to claim 1 or 2, characterised in that $R^9$ denotes alkyl having 1 to 4 C atoms, preferably methyl or ethyl.

4. Process according to one or several of claims 1 to 3, characterised in that the amine HZ is selected in such a way that, together with the carbonyl group of the ester II, it forms a radical $R^1$, which contains an -NH- grouping.

5. Process according to one or several of claims 1 to 4, characterised in that the amine HZ is selected in such a way that, together with the carbonyl group of the ester II, it forms a radical $R^1$ of the formulae

$-CONHR^3N(R^4)_2$, 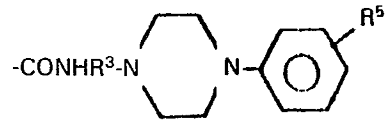

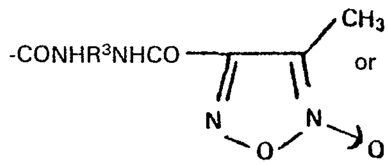 or $-CONHR^3OR^6$

wherein $R^3$ denotes $-CH_2CH_2-$ or $-CH_2CH_2CH_2-$ and $R^4$, $R^5$ and $R^6$ have the meanings given in claim 1.

6. Process for the preparation of agents for combating or preventing diseases of the cardiovascular system, characterised in that the 3,4- disubstituted 1,2,5-oxadiazole-2-oxides of formula I as well as pharmacologically acceptable acid addition compounds thereof

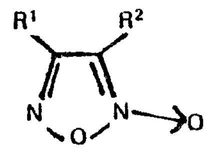

are prepared according to claim 1 and the compounds of formula I thus obtained or pharmacologically acceptable acid addition compounds thereof are converted, by mixing with pharmaceutically permissible excipients and, if appropriate, additives, into a form suitable for administering and combating or preventing diseases of the cardiovascular system. tem.